Europäisches Patentamt

European Patent Office

Office européen des brevets

(11) Veröffentlichungsnummer: **0 114 351**
**B1**

(12) # EUROPÄISCHE PATENTSCHRIFT

(45) Veröffentlichungstag der Patentschrift:
26.07.89

(51) Int. Cl.⁴: **A 61 L 15/01,** D 21 H 5/12,
**C 08 L 89/06**

(21) Anmeldenummer: **83112861.6**

(22) Anmeldetag: **21.12.83**

(54) Verfahren zur Herstellung eines Kollagen-Vlieses.

(30) Priorität: **27.12.82 DE 3248188**

(43) Veröffentlichungstag der Anmeldung:
**01.08.84 Patentblatt 84/31**

(45) Bekanntmachung des Hinweises auf die Patenterteilung:
**26.07.89 Patentblatt 89/30**

(84) Benannte Vertragsstaaten:
**AT CH DE FR IT LI**

(56) Entgegenhaltungen:
**EP-A- 0 023 607**
**FR-A- 2 328 786**
**US-A- 3 443 261**
**US-A- 3 742 955**
**US-A- 4 066 083**

(73) Patentinhaber: **BEHRINGWERKE Aktiengesellschaft,**
**Postfach 1140, D-3550 Marburg 1 (DE)**

(72) Erfinder: **Pâques, Eric Paul, Dr., Schmiedeacker 18,**
**D-3550 Marburg (DE)**
Erfinder: **Fuhge, Peter, Dr., Mühlenstrasse 8,**
**D-3551 Lahntal 2 (DE)**

(74) Vertreter: **Meyer-Dulheuer, Karl-Hermann, Dr. et al,**
**HOECHST Aktiengesellschaft Zentrale Patentabteilung**
**Postfach 80 03 20, D-6230 Frankfurt/Main 80 (DE)**

**Beschreibung**

Die Erfindung betrifft ein Verfahren zur Herstellung eines nativen und biologisch aktiven Kollagen-Vlieses aus menschlichen Plazenten.

Kollagen ist ein faserförmiges Protein, das hauptsächlich in Haut, Sehnen, Knochen und Bindegewebe von Menschen und Tieren vorkommt. Kollagenfasern der Gefäßwand bewirken die Adhäsion und die Aggregation von Thrombozyten und beeinflussen dadurch die Blutstillung.

Die Verwendung von Kollagenmaterialien zur Beschleunigung der Wundheilung ist bekannt. Kollagen führt zu einer deutlichen Steigerung der Wundfestigkeit und verstärkt das Fibroblasten-Wachstum.

Die Herstellung von Kollagen-Präparaten aus tierischem oder humanem Ausgangsmaterial ist bereits beschrieben worden. In der europäischen Patentanmeldung 0 023 607 wird ein Verfahren zur Gewinnung von biologisch aktivem Kollagen aus humaner Plazenta mit Hilfe einer Reihe von Wasch-Schritten und Pepsin-Abbau beschrieben. Weiterhin sind Verfahren zur Herstellung von Kollagen-Vliesen auch in der europäischen Patentanmeldung 0 042 253, in US-PS 3 471 598 und 3 823 212, DE-PS 2 943 520 sowie DE-OS 2 734 503 und 2 348 685 beschrieben. Die so hergestellten Produkte sind jedoch bei ihrer therapeutischen Anwendung nicht voll befriedigend, da sie nicht gleichzeitig alle erwünschten physikalischen und biologischen Eigenschaften aufweisen.

Es wurde überraschenderweise gefunden, daß durch Änderung des Verfahrens von EP-OS 0 023 607 und die Behandlung des kollagenhaltigen Materials mit einer Zitronensäurelösung eine stärkere Plättchen-aggregierende Wirkung sowie eine Verringerung einer Verunreinigung mit Hepatitis Virus B-Antigen erreicht wird. Gleichzeitig können mit der auf diese Weise hergestellten Kollagenlösung überraschenderweise Kollagenvliese mit optimalen biologischen und physikalischen Eigenschaften produziert werden.

Gegenstand der Erfindung ist deshalb ein Verfahren zur Herstellung eines Kollagenvlieses durch Homogenisieren und Blutfreiwaschen eines kollagenhaltigen Materials, Behandeln mit einer 0,5–4 molaren neutralen Salzlösung und einer 0,5–2 molaren Zitronensäurelösung von pH 1–4, Abbau mit Pepsin, Fällen des Kollagens mit NaCl, Auflösen in schwach gepufferter Lösung von pH 5, Dialysieren der Lösung gegen Wasser, Suspendieren der ausgefällten Kollagenfibrillen und Gefriertrocknen.

Gegenstand der Erfindung ist besonders ein solches Verfahren, worin nach dem Abbau mit Pepsin gegebenenfalls mit einem Ionenaustauscher behandelt und die Kollagenfibrillen mit einem Vernetzungsmittel behandelt werden.

Ein bevorzugtes Verfahren besteht beispielsweise darin, daß zerkleinerte und blutfrei gewaschene Plazenten mit einer 0,5–4 molaren Neutralsalzlösung, vorzugsweise einer 2 mol/l Natriumchlorid-Lösung, und einer 0,5–2 molaren Zitronensäurelösung von pH 1–4, vorzugsweise einer 1 mol/l Zitronensäurelösung von pH 2, gewaschen und mit Pepsin abgebaut wird. Gegebenenfalls kann die erhaltene Lösung zur Entfernung nieder- und hochmolekularer Verunreinigungen z.B. mit einem ionenaustauschenden Harz behandelt werden. Das Verfahren besteht außerdem darin, daß der Niederschlag einer Fällung mit Kochsalz in schwach gepufferter Lösung von pH 4–9, vorzugsweise pH 4–6 aufgenommen und gelöst, gegen Wasser, vorzugsweise 16 Stunden, dialysiert, der gebildete Niederschlag in wäßriger Lösung suspendiert, vorzugsweise bei pH 8, mit einem Vernetzungsmittel, vorzugsweise Formaldehyd, in einer Konzentration von 0,01–0,1 g/l, vorzugsweise 0,05 g/l, bei einer Temperatur von 4–37 °C, vorzugsweise 25 °C, 0,5 bis 24 Stunden, vorzugsweise 4 Stunden, behandelt und getrocknet, vorzugsweise gefriergetrocknet, wird.

In einer besonders bevorzugten Ausführungsform verfährt man so, daß man zerkleinerte und blutfrei gewaschene humane Plazenten mit einer schwach gepufferten Salzlösung von pH 7–9 und einer Konzentration von 0,5–4 mol/l, vorzugsweise mit 40–50 l/kg Plazentenmaterial einer schwach gepufferten 2 mol/l NaCl-Lösung, behandelt, den Niederschlag abtrennt, mit, vorzugsweise 40–50 l/kg Plazentenmaterial, Wasser wäscht, mit 4–50 l/kg Plazentenmaterial einer 0,5–2 mol/l, vorzugsweise 4 l/kg Plazentenmaterial 1 mol/l Zitronensäurelösung von pH 2, 0,5 bis 24 Stunden, vorzugsweise 2 Stunden, bei einer Temperatur von 2–25 °C, vorzugsweise 4 °C, behandelt, den abgetrennten Niederschlag mit Wasser wäscht, in einer wäßrigen Lösung einer Säure mit einem pK von 3–5, vorzugsweise mit 0,1 mol/l Essigsäure, aufnimmt, vorzugsweise mit Salzsäure, auf pH 2 bringt, 15 bis 30 Stunden, vorzugsweise 24 Stunden, bei 4–30 °C, vorzugsweise 25 °C, mit Pepsin behandelt, die Pepsinbehandlung wiederholt, gegebenenfalls die Lösung mit einem silikathaltigen Adsorptionsmittel, beispielsweise mit Dicalite® speed plus, versetzt, den Überstand abtrennt, diese Lösung durch Zugabe eines Puffers, vorzugsweise Tris-Salzsäure, auf einen pH-Wert von 7–11 bringt, gegebenenfalls mit einem Anionenaustauscher versetzt und diesen abtrennt, die Lösung durch Zugabe einer Säure, beispielsweise Salzsäure oder Essigsäure, auf einen pH-Wert von 2–3 bringt, durch Erhöhen der Ionenstärke, vorzugsweise durch Zugabe von Neutralsalz bis zu einer Konzentration von 0,6–1,3 mol/l, das Kollagen ausfällt, den abgetrennten Niederschlag in einer schwach gepufferten Lösung von pH 4–9, vorzugsweise pH 5 löst, die auf diese Weise erhaltene Kollagen-Lösung, vorzugsweise 16 Stunden bei 4 °C, gegen Wasser dialysiert, wobei sich Kollagenfibrillen ausscheiden, die Fibrillen homogenisiert, die Suspension, vorzugsweise bei pH 8, 0,5 bis 24 Stunden, vorzugsweise 4 Stunden, mit Formaldehyd in einer Endkonzentration von 0,01–0,1 g/l, vorzugsweise 0,05 g/l, bei 4–37 °C, vorzugsweise 25–37 °C, behandelt und zur Trockene bringt, vorzugsweise durch Einfrieren bei −60 °C bis −5 °C, vorzugsweise −40 °C, und Gefriertrocknen.

Die Kollagen-Vliese können, vorzugsweise durch γ-Bestrahlung, sterilisiert werden.

Ein auf diese Weise hergestelltes Kollagen-Vlies zeichnet sich durch Geruchlosigkeit, weiße Farbe, hohe Reißfestigkeit, gute Plastizität, Elastizität und Hydrophilie sowie die Neigung aus, die Plättchen-Aggregation auszulösen.

Durch die Gerbung mit Formalin wird die Gefahr einer Übertragung von Hepatitis-Non-A-Non-B-Viren vermieden (E. Tabor, R.J. Genty: J. of Infect. Dis. 142 (5), 767–770, 1980).

Trotz der Behandlung mit Formalin und einer Sterilisation mit γ-Strahlen besitzt das erfindungsgemäß hergestellte Vlies eine starke thrombozytenaggregierende Wirkung, was bei nach dem Stand der Technik hergestellten Kollagenvliesen nicht gefunden wurde.

Ein auf die beschriebene Weise hergestelltes Vlies eignet sich deshalb insbesondere für die Herstellung von Wundabdeckungen, die nicht nur zur Wundbefestigung und Verstärkung der Fibroblastenbildung, sondern auch zur Blutstillung, speziell auch bei Patienten mit besonderer Blutungsneigung (Hämophilie), beispielsweise nach einer Zahnextraktion, brauchbar sind.

Die Erfindung soll durch die nachstehenden Beispiele näher erläutert werden.

Beispiel 1

5 kg Plazentenrückstand, der durch Extraktion von blutfrei gewaschenen und zerkleinerten Plazenten mit isotonischer Kochsalzlösung und Abtrennung des Extrakts erhalten wird, wurde nochmals zerkleinert, abzentrifugiert und mit 250 l 0,05 mol/l Tris/HCl-Puffer, pH 7,4, enthaltend 2 mol/l NaCl, extrahiert. Die Suspension wurde dekantiert, der Niederschlag dreimal mit 250 l Wasser bei 4°C gewaschen und die Suspension dekantiert. Danach wurde der Niederschlag in 250 l 1 mol/l Zitronensäure suspendiert und nach 1 Stunde der Überstand abdekantiert. Mit einem Homogenisator wurde der Niederschlag fein verteilt und mit 20 l Wasser bei 4°C gewaschen und zentrifugiert. Der Niederschlag wurde in 20 l 0,1 mol/l Essigsäure aufgenommen und unter Rühren mit 1 n Salzsäure auf einen pH-Wert von 2 gebracht. 2 g Pepsin (110 Anson-Einheiten pro mg) wurden zugegeben und 24 Stunden bei einer Temperatur von 25°C gerührt. Diese Lösung wurde mit frischem Pepsin versetzt und unter denselben Bedingungen weitergerührt. Nach Zugabe von 40 g/l Dicalite® wurde homogenisiert und abzentrifugiert. Der Überstand wurde durch Zugabe von Tris auf pH 8,0 gebracht und mit 50 g/l Dowex® 2 × 8 eine Stunde gerührt, abzentrifugiert und auf pH 2 gestellt. Die Lösung wurde mit festem NaCl auf eine Konzentration von 0,2 mol/l gebracht. Nach zweistündigem Rühren wurde zentrifugiert und der Überstand verworfen. Das Präzipitat wurde in 10 l Wasser aufgenommen, mit Essigsäure auf pH 5 gestellt und gegen Wasser dialysiert, wobei Kollagen in Form weißer, geruchloser Fibrillen ausfiel.

Beispiel 2

Wie Beispiel 1, wobei der Plazenta-Rückstand 18 Stunden mit 25 l 0,5 mol/l Zitronensäure extrahiert wurde anstelle der dort angegebenen Zitronensäurebehandlung.

Beispiel 3

Wie Beispiele 1 und 2, wobei anstatt Tris-Puffer eine 2 mol/l Natrium-Chlorid-Lösung zur ersten Extraktion verwendet wurde.

Beispiel 4

Eine Kollagen-Suspension nach Beispiel 1, 2 oder 3 und eine nach EP-OS 0 023 607 hergestellte Kollagen-Suspension wurden in gleicher Konzentration in einem Aggregometer nach Born mit Schreiber (Fa. Braun, Melsungen, BRD) getestet. Dazu wurde 1 ml plättchenreiches Citratplasma in die Küvette gefüllt und Photometer und Schreiber auf 0% Transmission eingestellt. Unter Rühren wurde der Temperaturausgleich auf 37°C abgewartet. Dann wurde die jeweilige Kollagensuspension zugegeben und die Transmissionszunahme gemessen, die durch die Plättchenaggregation verursacht wird. Es wurde eine 10fach höhere Aktivität des nach dem Beispiel 1, 2 oder 3 erhaltenen Kollagens im Vergleich mit dem Kollagen des Standes der Technik gefunden.

Literatur: Born G.W.R., J. Physiol. (London) 162, 67 (1962).

Beispiel 5

Das erfindungsgemäße Verfahren wurde mit Zusätzen von ausreichenden HBsAg-Mengen durchgeführt, um die Eignung dieses Verfahrens bezüglich der Entfernung von HBsAg zu prüfen.

| Schritt | HBsAg (μg) zugesetzte Menge | restliche Menge | Abreiche-rungsfaktor |
|---|---|---|---|
| NaCl-Waschung ↓ | 9.000 | | |
| Überstand der 2. Wasser-Waschung | – | 82 | 110 |
| Zitronensäure-Waschung ↓ | 900 | – | |
| Überstand der 3. Wasser-Waschung | – | 7 | 129 |
| Pepsin-Abbau ↓ | 1.000 | – | |
| Kollagen-Lösung | – | 58 | 17 |

Es wurde ein Abreicherungfaktor von etwa 240 000 gefunden.

## Beispiel 6

300 ml Portionen einer 8–12 g/l Kollagenfibrillen enthaltenden Suspension von pH 5 wurden homogenisiert, auf pH 8 gebracht, mit Formaldehyd in einer Konzentration von 0,05 g/l 4 Stunden bei 25 °C behandelt. Die Suspensionen wurden bei –40 °C eingefroren und zu Vliesen gefriergetrocknet.

## Beispiel 7

Kollagen-Vliese wurden wie in Beispiel 6 hergestellt. Allerdings wurde die Vernetzung mit Formalin bei unterschiedlichen pH-Werten von 4–10 durchgeführt. Die Kollagen-Vliese wurden auf ihre Reißfestigkeit getestet.

| Gerbung bei pH | Reißfestigkeit (g) |
|---|---|
| 4 | 51 |
| 6 | 67 |
| 7 | 99 |
| 8 | 166 |
| 10 | 120 |

## Beispiel 8

Erfindungsgemäß erhaltenes Kollagen-Vlies und zwei herkömmliche, käufliche Kollagen-Vliese wurden, wie in Beispiel 4 beschrieben, geprüft, wobei die Kollagenlösung durch die gleiche Menge Kollagen-Vlies ersetzt wurde. Nur das nach dem erfindungsgemäßen Verfahren hergestellte Vlies löste eine Plättchenaggregation aus.

## Beispiel 9

Kollagen-Vliese wurden wie in Beispiel 5 hergestellt. Allerdings wurde das kochsalzgefällte Kollagen in einer schwach gepufferten Lösung von pH 4,0–9,0 gelöst und gegen Wasser dialysiert. Die hergestellten Kollagen-Vliese wurden auf ihre physikalischen und biologischen Eigenschaften getestet. Die besten Ergebnisse wurden bei der bei pH 5,0 gelösten Kollagen-Lösung beobachtet (siehe Tabelle).

| pH | Wasseraufnahme-geschwindigkeit (sec) | Wasseraufnahme-menge (g) | Reißfestig-keit (g) | Elastizität |
|---|---|---|---|---|
| 4,0 | 25,0 | 1,1 | <30 | 23 |
| 5,0 | 1,8 | 1,9 | 216 | 5,5 |
| 6,0 | 1,9 | 1,8 | 110 | 7,5 |
| 7,0 | 2,3 | 0,94 | <30 | 17,7 |
| 8,0 | 11,5 | 1,0 | <30 | >180 |
| 9,0 | 30,0 | 0,92 | <30 | >180 |

## Patentansprüche

1. Verfahren zur Herstellung eines Kollagenvlieses durch Homogenisieren und Blutfreiwaschen eines kollagenhaltigen Materials, Behandeln mit einer 0,5–4 molaren neutralen Salzlösung und einer 0,5–2 molaren Zitronensäurelösung von pH 1–4, Abbau mit Pepsin, Fällen des Kollagens mit NaCl, Auflösen in schwach gepufferter Lösung von pH 5, Dialysieren der Lösung gegen Wasser, Suspendieren der ausgefällten Kollagenfibrillen und Gefriertrocknen.

2. Verfahren nach Anspruch 1, dadurch gekennzeichnet, daß die suspendierten Kollagenfibrillen mit einem Vernetzungsmittel in einer Konzentration von 0,01 bis 0,1 g/l bei einer Temperatur von 4–37 °C 0,5 bis 24 Std. behandelt und getrocknet werden.

3. Verfahren nach Anspruch 2, dadurch gekennzeichnet, daß das Vernetzungsmittel Formaldehyd ist.

4. Verfahren nach Anspruch 1–3, dadurch gekennzeichnet, daß das kollagenhaltige Material Plazenta ist.

5. Kollagenvlies erhältlich nach dem Verfahren nach einem der Ansprüche 1 bis 3, dadurch gekennzeichnet, daß das kollagenhaltige Material menschliche Plazenta ist.

6. Kollagenvlies nach Anspruch 5 oder erhalten nach einem der Ansprüche 1 bis 4 als Wundabdeckung.

## Claims

1. A process for the production of a bonded collagen fiber sheet by homogenizing a collagen-containing material and washing it free of blood, treating with a 0.5–4 molar neutral salt solution and a 0.5–2 molar citric acid solution of pH 1–4, degrading with pepsin, precipitating the collagen with NaCl, dissolving in a weakly buffered solution of pH 5, dialyzing the solution against water, suspending the precipitated collagen fibrils and freeze-drying.

2. The process as claimed in claim 1, wherein the suspended collagen fibrils are treated with a crosslinking agent in a concentration of 0.01 to 0,1 g/l at a temperature of 4–37 °C for 0.5 to 24 h and are dried.

3. The process as claimed in claim 2, wherein the crosslinking agent is formaldehyde.

4. The process as claimed in claim 1–3, wherein the collagen-containing material is placenta.

5. A bonded collagen fiber sheet obtainable by the process as claimed in one of claims 1 to 3, .wherein the collagen-containing material is human placenta.

6. A bonded collagen fiber sheet as claimed in claim 5 or obtained as claimed in one of claims 1 to 4 as a covering for wounds.

## Revendications

1. Procédé de préparation d'un tampon de collagène par homogénéisation et lavage jusqu'à absence complète de sang d'une matière contenant du collagène, traitement par une solution de sel neutre 0,5–4 molaire et par une solution d'acide citrique 0,5–2 molaire à pH 1–4, dégradation par la pepsine, précipitation du collagène par NaCl, dissolution dans une solution faiblement tamponnée à pH 5, dialyse de la solution contre de l'eau, mise en suspension des fibrilles de collagène précipitées et lyophilisation.

2. Procédé suivant la revendication 1, caractérisé en ce que les fibrilles de collagène en suspension sont traitées par un agent de réticulation à une concentration de 0,01 à 0,1 g/l à une température de 4 à 37 °C pendant 0,5 à 24 heures, et séchées.

3. Procédé suivant la revendication 2, caractérisé en ce que l'agent de réticulation est le formaldéhyde.

4. Procédé suivant les revendications 1 à 3, caractérisé en ce que la matière contenant du collagène est du placenta.

5. Tampon de collagène pouvant être obtenu par le procédé suivant l'une des revendications 1 à 3, caractérisé en ce que la matière contenant du collagène est du placenta humain.

6. Tampon de collagène suivant la revendication 5 ou obtenu suivant l'une des revendications 1 à 4 pour panser les plaies.